Europäisches Patentamt

(19) European Patent Office

Office européen des brevets

(11) Publication number: 0 396 777

A1

(12) EUROPEAN PATENT APPLICATION
published in accordance with Art.
158(3) EPC

(21) Application number: 89912495.2

(51) Int. Cl.5: A61K 37/66

(22) Date of filing: 13.11.89

(86) International application number:
PCT/JP89/01156

(87) International publication number:
WO 90/05535 (31.05.90 90/12)

(30) Priority: 14.11.88 JP 288591/88

(43) Date of publication of application:
14.11.90 Bulletin 90/46

(84) Designated Contracting States:
CH DE FR GB LI NL

(71) Applicant: OTSUKA PHARMACEUTICAL CO.,
LTD.
9, Kandatsukasacho 2-chome
Chiyoda-ku Tokyo 101(JP)

(72) Inventor: ISHIZUE, Yoshihiro
200-3, Takase Kamiita-cho Itano-gun
Tokushima 771-13(JP)
Inventor: KIMURA, Yuzo
4-3-10, Minamisho-machi Tokushima-shi
Tokushima 770(JP)
Inventor: TODA, Masafumi
463-10, Kagasuno Kawauchi-cho
Tokushima-shi
Tokushima 770(JP)
Inventor: MASUDA, Yoshito
463-10, Kagasuno Kawauchi-cho
Tokushima-shi
Tokushima 770(JP)

(74) Representative: Hansen, Bernd, Dr.
Dipl.-Chem. et al
Hoffmann, Eitle & Partner Patent- und
Rechtsanwälte Arabellastrasse 4
D-8000 München 81(DE)

(54) INTERFERON PREPARATION FOR NASAL ADMINISTRATION.

(57) The invention provides preparation for nasal administration which contains a pharmacologically effective amount of interferon and a fatty acid ester of sugar.

DESCRIPTION

Interferon Preparation for Nasal Administration

Technical Field

The present invention relates to interferon preparations for nasal administration, and more particularly to an interferon preparation for nasal administration which is applied by dropping or spraying into the nasal cavity in the form of a powder or liquid.

Background Art

Interferons (IFNs) are bioactive substances which display various clinical effects such as antiviral effect, antitumor effect and immunological enhancement effect and so have been developed as antiviral agents, anticancer agents, etc. However, such pharmaceutical preparations which contain IFNs as active components are limited to injectable forms such as intravenous injection, intramuscular injection and subcutaneous injection, since IFNs are polypeptides to be immediately inactivatd by protease, etc. and are difficult to absorb through the gastrointestinal tract, skin and mucosa due to their large molecular weight.

Administration of such injections not only gives severe pain to patients but also makes home care difficult, hence very inconvenient. Therefore, it is desired in this field to research and develop more

- 2 -

convenient dosage forms other than injections. Preparations for nasal administration are exemplified as such dosage forms and disclosed in Unexamined Japanese Patent Publication No. 207226/87, etc. However, the absorbefacients themselves used in such preparations largely stimulate nasal mucosa and thus have not been yet usable for nasal preparation in spite of their relatively large IFN-absorption promoting effect.

## Disclosure of Invention

The present invention provides IFN preparations for nasal administration which can be effectively absorbed through the nasal mucosa and have little or no likelihood of stimulating the nasal mucosa for use with high safety.

The present invention relates to preparations for nasal administration comprising an IFN in a pharmacologically effective amount and a sucrose-fatty acid ester.

The inventors have found that, if sucrose fatty acid esters are used as absorbefacients together with IFN, IFN can be very efficiently absorbed through the nasal mucosa, and that the IFN preparation thus obtained for nasal administration does not substantially impair the nasal mucosa but is usable with extremely high safety to the living body. The present invention has been accomplished based on the above novel findings.

In the nasal preparation of the present invention, a solution or powder containing an active substance is inhaled into the nasal cavity using an appropriate instrument such as a spray bomb, aerosol bomb or nebulizer aspirator, whereby the active substance is adhered to and absorbed through the nasal mucosa. The present invention provides a nasal preparation comprising an IFN as the above-mentioned active substance, and more particularly a nasal preparation which contains a specific absorbefacient to efficiently promote the absorption of the IFN and which hardly stimulates the nasal mucosa substantially.

The IFN to be used in the present invention is at least one compound selected from the group comprising interferon-α (IFN-α), interferon-β (IFN-β) and interferon-γ (IFN-γ), which are already known. The method for preparing the present preparation for nasal administration is applicable to various bioactive substances as well as IFNs. Such bioactive substances include hormones such as insulins, calcitonins, luteohormone-releasing hormones, growth hormones, oxytocins, vasopressins, desmopressins; vaccines such as influenzae vaccines, hepatitis B vaccines, whooping cough vaccines; enzyme proteins such as urokinases, tissue plasminogen activators; bioactive proteins such as interleukins, tumor necrosis factors (TNF), immunoglobulins, blood coagulation factors VIII; etc.

The absorbefacient to be used in the present invention is at least one sucrose fatty acid ester. Such sucrose fatty acid esters are preferably selected from those having 8 to 22 carbon atoms in their fatty acid moiety and more preferably those having 12 to 18 carbon atoms in their fatty acid moiety. These sucrose fatty acid esters are known and any of them can be used. Fatty acids constituting these esters may be saturated or unsaturated and straight- or branched-chain ones, and are particularly preferably straight-chain saturated fatty acids.

The amounts of IFN and sucrose fatty acid ester (absorbefacient) to be contained in the present nasal preparation are somewhat variable and can be appropriately decided depending on the kind of these components. IFN is generally used in a concentration of about $10^3$ to about $10^9$ IU per preparation. Sucrose fatty acid esters are used generally in a concentration of about 0.05 to about 10 w/v%, preferably about 0.1 to about 5.0 w/v%.

The nasal preparation of the present invention can be prepared by conventional methods suitable for the respective dosage forms. For example, preparations of the liquid type can be formulated by properly mixing IFN and sucrose fatty acid ester (absorbefacient) and dissolving, emulsifying or dispersing the mixture in water or in an aqueous solution containing an organic solvent which is generally acceptable for use in nasal preparations. If desired, various pharmaceutically acceptable additives can be added which include, for example, buffers, stabilizing agents, preservatives, isotonic agents and viscosity increasing agents. Preparations of the solid type such as powder can also be prepared by a conventional method of pulverization.

The nasal preparation of the present invention thus obtained, like conventional nasal preparations, is intranasally given to the patient in an effective dose to exhibit the desired pharmacological effect. Particularly the present preparation can be administered in a relatively large amount because it is markedly less stimulant to the nasal mucosa, whereas the preparation displays a sufficient pharmacological effect even in a relatively small amount, since it is rendered highly absorbable by the addition of the specific absorbefacient.

### Examples

The present invention will be described in more detail with reference to examples, comparative examples and test examples, but is not limited by these examples.

1) IFN-$\alpha$ standard solution (manufactured by Hayashibara Biochemical Laboratories):

A standard solution wherein IFN derived from human BALL-1 cells (specific activity of 50000000 IU/mg) is dissolved in an amount of 30000000 IU/ml in a phosphate buffer (pH 6) containing human serum albumin (0.1 w/v%) as a stabilizer.

2) IFN-β standard preparation (manufactured by Toray Industries, Inc.):

A lyophilized preparation containing 3000000 IU per vial of IFN derived from human fibloblasts (specific activity of 10000000 IU/mg) and 9 mg per vial of human serum albumin as a stabilizer.

3) IFN-γ standard solution (manufactured by Hayashibara Biochemical Laboratories):

A standard solution wherein IFN derived from human HBL-38 cells (specific activity of 10000000 IU/mg) is dissolved in an amount of 30000000 IU/ml in a phosphate buffer (pH 7.2) containing human serum albumin (0.1 w/v%) as a stabilizer.

4) Sucrose fatty acid esters:

Sucorose lauric acid ester, sucrose palmitic acid ester, sucrose stearic acid ester and sucrose oleic acid ester (all are manufactured by Mitsubishi Chemical Food Co.,).

Example 1

A 50 mg quantity of sucrose lauric acid ester was dissolved in 5 ml of IFN-α standard solution, and D-mannitol was added thereto. The mixture was adjusted to osmotic ratio of 2 and lyophilized to afford a nasal preparation according to the present invention.

This preparation is given as dissolved in 10 ml of water at use.

## Example 2

A 5 ml portion of 1 w/v% sucrose palmitic acid ester solution was added to 5 ml of IFN-α solution, followed by isotonization with D-mannitol. A nasal preparation according to the present invention was thus obtained.

## Example 3

The lyophilized powder of IFN-α standard solution was added to 0.5 w/v% sucrose stearic acid ester solution to prepare a solution of 15000000 IU/ml. This solution was isotonized with D-mannitol, giving a nasal preparation of the invention.

## Example 4

A 5 ml portion of 1 w/v% sucrose stearic acid ester solution was added to 5 ml of IFN-γ standard solution, followed by isotonization with D-mannitol. A nasal preparation of the present invention was thus obtained.

- 8 -

### Example 5

A 0.2 ml portion of 0.5 w/v% sucrose stearic acid ester was added to IFN-β standard solution, giving a nasal preparation of the present invention.

### Example 6

A 5 ml portion of 1 w/v% sucrose oleic acid ester solution was added to 5 ml of IFN-α solution, and the mixture was isotonized with D-mannitol, giving a nasal preparation of the invention.

### Example 7

A 50 mg quantity of sucrose stearic acid ester was dissolved in 5 ml of IFN-α standard solution, and the solution was isotonized with D-mannitol, followed by lyophilization. A nasal preparation was thus obtained according to the invention.

This preparation is to be given as dissolved in 5 ml of water.

### Comparative Example 1

A 5ml portion of water was added to 5 ml of IFN-α standard solution, and the mixture was isotonized with D-mannitol to give a nasal preparation for comparison.

### Comparative Example 2

A 50 mg quantity of polyoxyethylene (9) lauryl ether (BL-9EX) was dissolved in 5 ml of IFN-α standard solution. The solution was isotonized with sodium chloride, giving a nasal preparation for comparison.

### Comparative Example 3

A 50 mg quantity of sodium glycocholate was dissolved in 5 ml of IFN-α standard solution. The solution was isotonized with sodium chloride, giving a nasal preparation for comparison.

### Comparative Example 4

A 50 mg quantity of saponin was dissolved in 5 ml of IFN-α standard solution. The solution was isotonized with sodium chloride, giving a nasal preparation for comparison.

### Test Example 1

### IFN-absorption promoting effect

Male Sprague-Dawley rats (weighing 200-300 g) were anesthetized intraperitoneally with pentobarbital (50 mg/kg). Subsequently, the cervix was opened and a polyethylene tube was inserted into the trachea to rescue the airway. A part of the esophagus was opened, and a polyethylene tube whose end was stoppered was inserted thereinto in the direction of the postnaris to stop the postnaris.

Each of the preparations of the above examples and reference examples was intranasally administered in a dose of 0.1 ml/kg. Blood was then drawn periodically from jugular to determine antiviral activity of IFN in serum.

The result is shown in Table 1.

Table 1

| | Change in serum IFN levels (IU/ml) | | | | | |
|---|---|---|---|---|---|---|
| | 0 min | 15 min | 30 min | 60 min | 90 min | 120 min |
| Example 1 | N.D. | 548 | 622 | 252 | 156 | 127 |
| Example 2 | N.D. | 180 | 375 | 316 | 228 | 232 |
| Example 3 | N.D. | 349 | 376 | 400 | 369 | 138 |
| Example 4 | N.D. | 43 | 114 | 138 | 108 | 71 |
| Example 5 | N.D. | 29 | 59 | 113 | 83 | 75 |
| Example 7 | N.D. | 880 | 1102 | 783 | 436 | 271 |
| Comparative Example 1 | N.D. | N.D. | N.D. | N.D. | N.D. | N.D. |
| Comparative Example 2 | N.D. | 402 | 548 | 488 | 388 | 372 |
| Comparative Example 3 | N.D. | 235 | 271 | 199 | 86 | 43 |
| Comparative Example 4 | N.D. | 374 | 300 | 226 | 213 | 210 |

In the above table, N.D. represents "not detectable". Each value given is the mean of three test values.

The result shows that with all the nasal preparations of the present invention comprising a sucrose $C_{12-18}$-fatty acid ester, IFN is absorbed in a larger amount than in the case with the comparative preparation containing only IFN (Comparative example 1) and is absorbed in as great an amount as or in a larger amount

- 11 -

than in the case of the comparative preparations comprising other conventional absorbefacient (Comparative Examples 2 to 4).

## Test Example 2

### Protein Dissolution by Absorbefacient

The absorbefacients used were tested for the stimulant effect on the nasal mucosa by determining protein dissolution according to the following in situ recirculation technique.

Male Sprague-Dawley rats (weighing about 200-300 g) were anesthetized intraperitoneally with pentobarbital (50 mg/kg). Subsequently, the cervix was opened and a polyethylene tube was inserted into the trachea to rescue the airway. A part of the esophagus was opened, and a polyethylene tube whose end was stoppered was inserted thereinto in the direction of the postnaris to connect the tube to the postnasal. The preparations of the examples and comparative examples were diluted 2-fold with physiological saline. (In these dilutions, the absorbefacient was contained in a constant concentration of 0.5 w/v% (minimum concentration for efficient absorption of IFN.) A 20 ml portion of each dilution was transfused at a flow rate of 1 ml/min using a transfusion pump and perfused through the nasal cavity for 30 minutes. A part of the perfusate was sampled 15 minutes

- 12 -

and 30 minutes after starting the perfusion to determine protein dissolution in the perfusion liquid. The degree of stimulating effect on the nasal mucosa was thus evaluated.

The result is shown in Table 2.

Table 2

|  | Protein Dissolution ($\mu$g/0.1 ml) | |
|---|---|---|
|  | 15 minutes | 30 minutes |
| Example 1 | 50.3 | 88.2 |
| Example 2 | 23.5 | 32.4 |
| Example 3 | 17.4 | 30.8 |
| Example 6 | 15.5 | 28.2 |
| Comparative Example 1 | 13.0 | 19.8 |
| Comparative Example 2 | 51.9 | 91.8 |
| Comparative Example 3 | 54.2 | 119.4 |
| Comparative Example 4 | 114.7 | 192.7 |

The result reveals that all the nasal preparations of the present invention comprising sucrose $C_{12-18}$ fatty acid ester causes slightly greater stimulation than the comparative preparation containing only IFN (Comparative example 1) but are much less stimulant than the comparative preparations comprising other conventional absorbefacient (Comparative examples 2 to 4).

- 13 -

CLAIMS:

1. A preparation for nasal administration comprising an IFN in a pharmacologically effective amount and a sucrose fatty acid ester.

2. A preparation according to claim 1 wherein the sucrose fatty acid ester is an ester of $C_{8-22}$ fatty acids.

3. A preparation according to claim 1 wherein the sucrose fatty acid ester is an ester of $C_{12-18}$ fatty acids.

4. A preparation according to claim 1 wherein the sucrose fatty acid ester is contained in a concentration of 0.05-10 w/v%.

5. A preparation according to claim 1 wherein the sucrose fatty acid ester is contained in a concentration of 0.01-5.0 w/v%.

# INTERNATIONAL SEARCH REPORT

International Application No  PCT/JP89/01156

## I. CLASSIFICATION OF SUBJECT MATTER (if several classification symbols apply, indicate all) [5]

According to International Patent Classification (IPC) or to both National Classification and IPC

Int. Cl$^5$    A61K37/66

## II. FIELDS SEARCHED

### Minimum Documentation Searched [7]

| Classification System | Classification Symbols |
|---|---|
| IPC | A61K37/66, 9/12, 9/14, A61K47/26 |

### Documentation Searched other than Minimum Documentation to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | JP, A, 59-163313 (Teijin Ltd.), 14 September 1984 (14. 09. 84), Claim & EP, A, 122036 | 1 - 5 |
| A | JP, A, 62-281813 (Delaware Chemicals Corp.), 7 December 1987 (07. 12. 87), Claim & EP, A, 242643 | 1 - 5 |
| A | JP, A, 63-115822 (Teijin Ltd.), 20 May 1988 (20. 05. 88), Claim (Family: none) | 1 - 5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| January 12, 1990 (12. 01. 90) | January 22, 1990 (22. 01. 90) |
| International Searching Authority | Signature of Authorized Officer |
| Japanese Patent Office | |

Form PCT/ISA/210 (second sheet) (January 1985)